# EUROPEAN PATENT SPECIFICATION

(11) **EP 2 263 676 B1**
(45) Date of publication and mention of the grant of the patent: **31.08.2011**
(21) Application number: 10153974.0
(22) Date of filing: 18.02.2010
(51) Int. Cl.: A61K 31/58, A61P 31/12

(54) **Compositions for inhibiting or treating Hepatitis C viruses**
Zusammensetzungen zur Hemmung oder Behandlung von Hepatitis-C-Viren
Compositions pour l'inhibition ou le traitement du virus de l'hépatite C

(30) Priority: 16.06.2009 TW 098120040; 28.10.2009 TW 098136423
(43) Date of publication of application: 22.12.2010
(73) Proprietor: Industrial Technology Research Institute, Hsinchu 31040 (TW)
(72) Inventor: Lee, Lain-Tze, 300, Hsinchu City (TW); Chang, Shau-Feng, 30069, Hsinchu City (TW); Tsai, Hui-Ping, Hsinchu City (TW); Kuo, Zong-Keng, 23548, Zhonghe City (TW); Chung, Chen-Fang, 30353, Hsinchu County (TW)
(74) Representative: Fuchs

(56) References cited:
- DATABASE WPI Week 200437 Thomson Scientific, London, GB; AN 2004-390844 XP002600851 SHI Y.; WANG W; WANG Z: "Toosendanin used as anti-tumor medicine, is separated from phloem of toosendan and purified" & CN 1 476 829 A ((SHAN-N) SHANGHAI RES INST LIFE SCI CHINESE ACAD) 25 February 2004 (2004-02-25)

## Description

### Field of the Invention

The present invention relates to the use of an anti-hepatitis C composition, and in particular, relates to the use of a composition comprising a limonoid compound to inhibit hepatitis C viruses or to treat hepatitis C.

### Description of the Related Art

According to recent estimates, about 2-3 % of the world population is infected with hepatitis C and the number is increasing by 3-4 million patients every year. Presently, the only tested and approved anti-hepatitis C drug is α-interferon. Meanwhile, ribavirin has been used to enhance the anti-hepatitis C curative effect of α-interferon. However, use of both drugs induces serious side effects and results in drug-resistance in patients.

The basic illustrative structure of the limonoid compound comprises three 6-membered rings and a 5-membered ring connected to a furan ring by a single bond. The limonoid compounds mainly exist in plants of Rutaceae and Meliaceae. Toosendanin of limonoid compounds may be extracted from Melia toosendan Sieb. Et Zucc. Toosendanin is mainly used as a natural insecticide and is known to be capable of inducing apoptosis of human leukemia cells. Furthermore, toosendanin may also be extracted from chuanlianzi (fructus toosendan) which is mature, dried fruit of the Melia toosendan Sieb. Et Zucc and used as an herb for driving out roundworm and pinworm.

However, limonoid compounds being applied to effectively inhibit hepatitis C has yet to be disclosed.

The invention refers to the use of an anti-hepatitis C composition, comprising: an effective amount of limonoid compound, wherein the structure of the limonoid compound is shown as Structure (I): 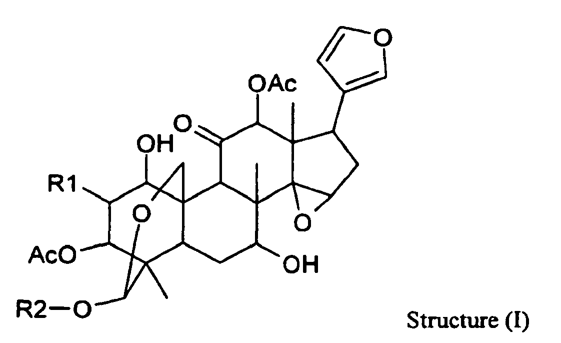 , where R1 comprises H or OAc and R2 comprises H or COCH(CH₃)₂; and a pharmaceutically acceptable carrier or salt, for inhibiting hepatitis C viruses or treating hepatitis C.

The invention also provides the use of a limonoid compound for preparing a drug for inhibiting hepatitis C viruses or treating hepatitis C, the structure of the limonoid compound is shown as Structure (I): 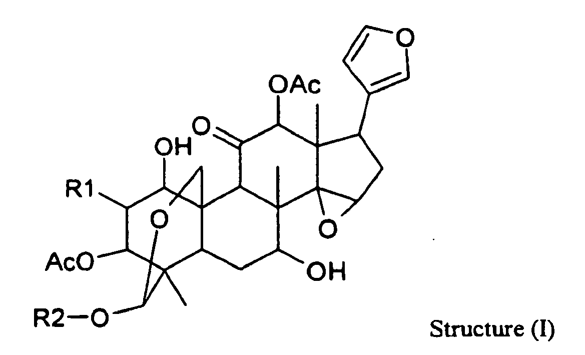 , where R1 comprises H or OAc and R2 comprises H or COCH(CH₃)₂.

A detailed description is given in the following embodiments with reference to the accompanying drawings.

The following description is of the best-contemplated mode of carrying out the invention. This description is made for the purpose of illustrating the general principles of the invention and should not be taken in a limiting sense. The scope of the invention is best determined by reference to the appended claims.

The invention provides a novel use of a composition comprising a limonoid compound as an anti-hepatitis C drug which is useful for inhibiting hepatitis C viruses or treating hepatitis C. In one embodiment, the used composition may comprise an effective amount of limonoid compound and a pharmaceutically acceptable carrier or salt, and a structure of the limonoid compound is shown as Structure (I):

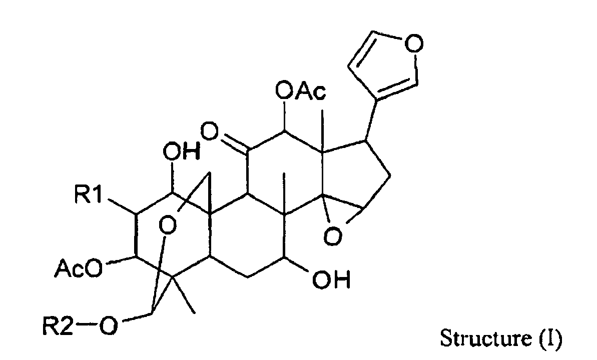

, where R1 comprises H or OAc and R2 comprises H or COCH(CH₃)₂.

Moreover, the limonoid compound may be extracted from a plant material, and the plant material may comprise Melia toosendan Sieb. Et Zucc. or Melia azedarach Linn.

The limonoid compound may comprise toosendanin or Trichilin H. Structures of toosendanin and Trichilin H are shown as Structures (II) and (III), respectively: 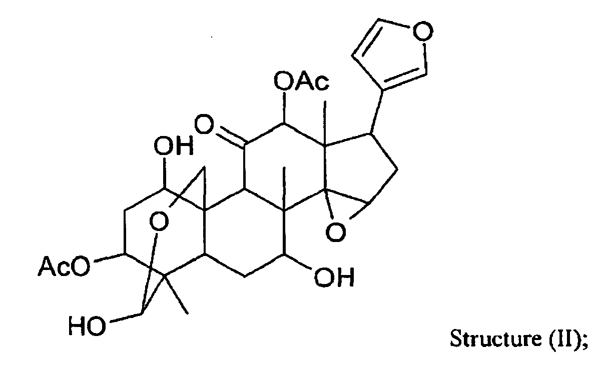 and

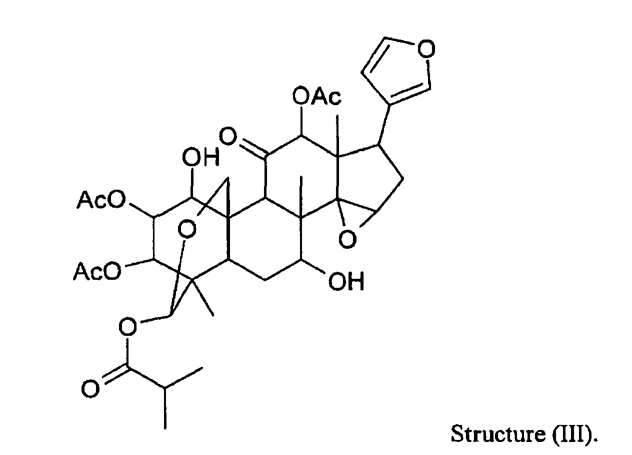

The HCV replicon system has been used worldwide as new drug development tool (V. Lohmann et al., 1999, Science. Vol. 285, 110-113 ; R. Bartenschlager, 2002, Nature Reviews/Drug Discovery. Vol. 1, 911-916 ; J. M. Vorlijk et al., 2003, Journal of Virological Methods. 110:201-209). The Huh-luc/neo-ET cell has I389luc-ubi-NS3-3'/ET replicon and could be used to monitor the replicative capability of the HCV. The Huh-luc/neo-ET is able to express a firefly luciferase-ubiquitin-neomycin fusion protein translated by the internal ribosomal entry site (IRES) of the hepatitis C virus and is able to express the hepatitis C viral nonstructural protein (NS3-5B) including protease, helicase and polymerase translated by the IRES of the encephalomyocarditis virus (EMCV). When the replication complex composed of the IRES of the hepatitis C virus or the nonstructural protein of the hepatitis C virus is influenced by a candidate, the effect of the candidate for inhibiting the activity of the hepatitis C virus repilcon can be estimated by determining the intensity of the firefly luciferase activity. The hepatitis C virus inhibition effect of the limonoid compound can be determined by determining the firefly luciferase activity expressed by the Huh-Iuc/noe-ET cell in presence of the limonoid compound.

In the Huh-Iuc/neo-ET cell, 50 % of the HCV replication inhibition concentration (IC₅₀) of the limonoid compound is at least less than about 0.5 µg/ml, preferably about 0.045 µg/ml. Furthermore, the effect window (EW), a ratio of 50% of the cell cytotoxic concentration (CC₅₀) to IC₅₀, of the limonoid compound is at least greater than about 100, preferably greater than about 2500.

In one embodiment, the limonoid compound to be used according to the invention may comprise toosendanin Structure of toosendanin is shown as Structure (II):

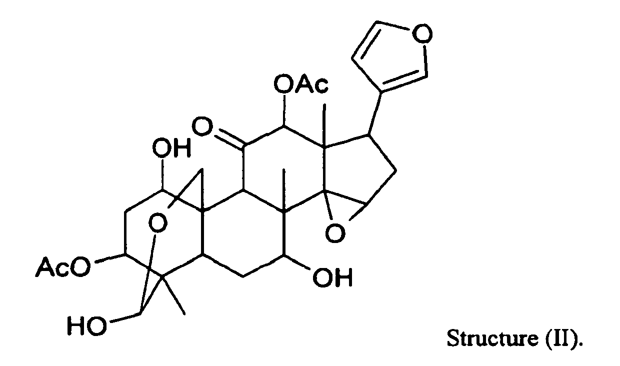

The toosendanin may be extracted from a plant material, and the plant material may comprise Melia toosendan Sieb. Et Zucc. or Melia azedarach Linn. In the Huh-luc/neo-ET cell, the IC₅₀ of the toosendanin is at least less than about 0.05 µg/ml, and the EW of the toosendanin is at least greater than about 2500.

In another embodiment, the limonoid compound may comprise Trichilin H. Structure of Trichilin H is shown as Structure (III):

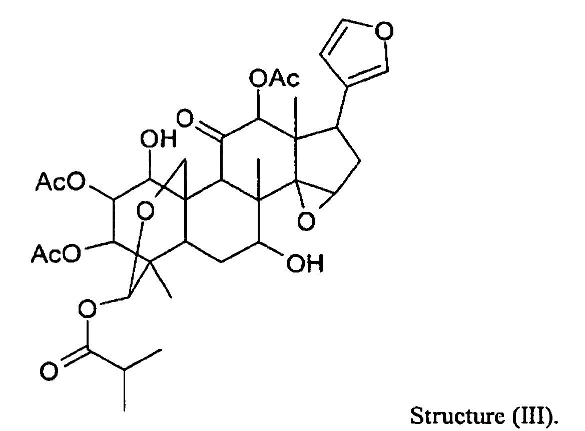

The Trichilin H may be extracted from a plant material, and the plant material may comprise Melia toosendan Sieb. Et Zucc. or Melia azedarach Linn. In the Huh-luc/neo-ET cell, the IC₅₀ of the Trichilin H is at least less than about 0.5 µg/ml, and the EW of the Trichilin H is at least greater than about 100.

A pharmaceutically acceptable carrier may comprise, but is not limited to, a solvent, a dispersion medium, a coating, an antibacterial and antifungal agent, or an isotonic and absorption delaying agent. The pharmaceutical composition can be formulated into dosage forms for different administration routes utilizing conventional methods.

The pharmaceutically acceptable salt may comprise, but is not limited to, inorganic cation salts including alkali metal salts such as sodium salt, potassium salt or amine salt, alkaline-earth metal salt such as magnesium salt or calcium salt, the salt containing bivalent or quadrivalent cation such as zinc salt, aluminum salt or zirconium salt. In addition, the pharmaceutically acceptable salt may also comprise organic salt including dicyclohexylamine salt, methyl-D-glucamine, and amino acid salt such as arginine, lysine, histidine, or glutamine.

The pharmaceutical composition may be administered orally, parenterally by an inhalation spray or via an implanted reservoir. The parenteral method may comprise subcutaneous, intracutaneous, intravenous, intramuscular, intraarticular, intraarterial, intrasynovial, intrasternal, intrathecal, and intraleaional, as well as infusion techniques.

An oral composition can comprise, but is not limited to, tablets, capsules, emulsions and aqueous suspensions, dispersions and solutions.

>

For the preparation of a drug used in accordance with the invention, an effective amount of limonoid compound is used as an active ingredient for the drug. Structure of the limonoid compound is shown as Structure (I):

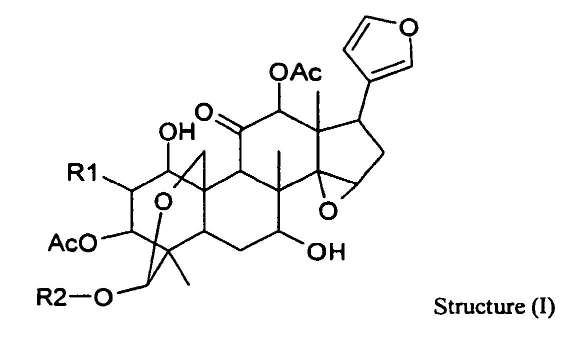

, where R1 comprises H or OAc and R2 comprises H or COCH(CH₃)₂.

The limonoid compound may be extracted from a plant material, and the plant material may comprise Melia toosendan Sieb. Et Zucc. or Melia azedarach Linn.

In addition, the limonoid compound may comprise toosendanin or Trichilin H. Structures of toosendanin and Trichilin H are shown as Structures (II) and (III), respectively: 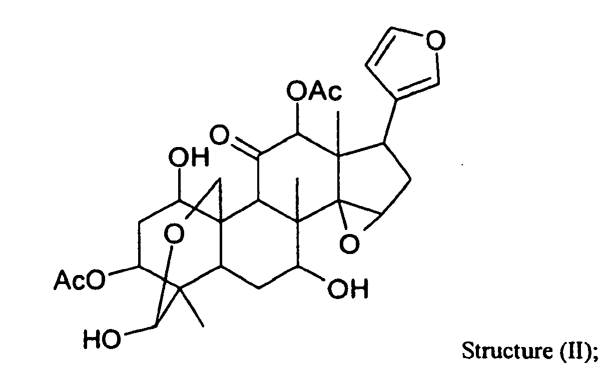 and

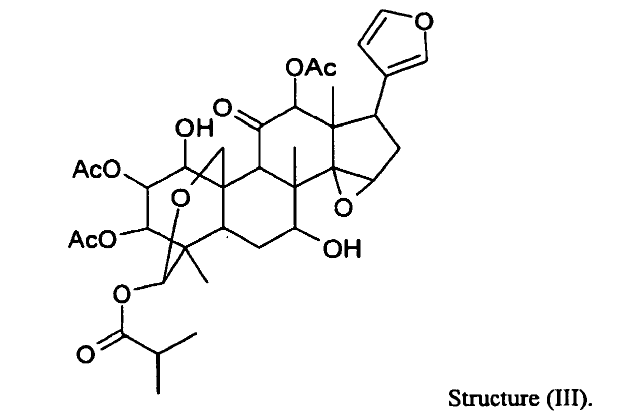

The use of the compositions according to the invention for treating hepatitis C may comprise administering an effective amount of an anti-hepatitis C composition to a patient in need.

The used anti-hepatitis C composition is described as above, which may comprise an effective amount of limonoid compound and a pharmaceutically acceptable carrier or salt.

Structure of the limonoid compound is shown as Structure (I):

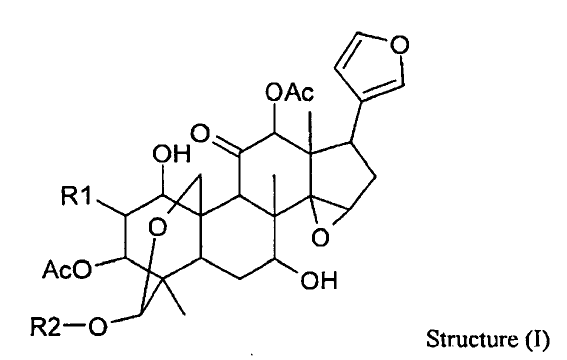

, where R1 comprises H or OAc and R2 comprises H or COCH(CH₃)₂.

The limonoid compound may be extracted from a plant material, and the plant material may comprise Melia toosendan Sieb. Et Zucc. or Melia azedarach Linn.

Moreover, the limonoid compound may comprise toosendanin or Trichilin H. Structures of toosendanin and Trichilin H are shown as Structures (II) and (III), respectively: 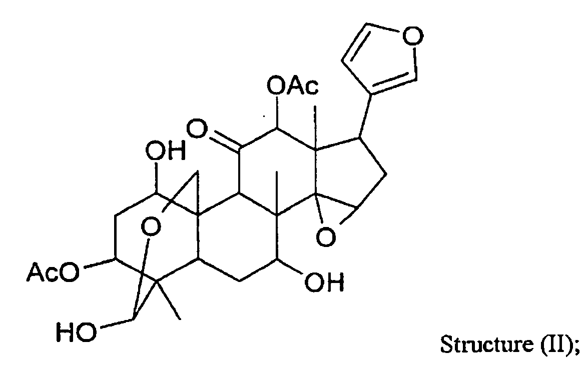 and

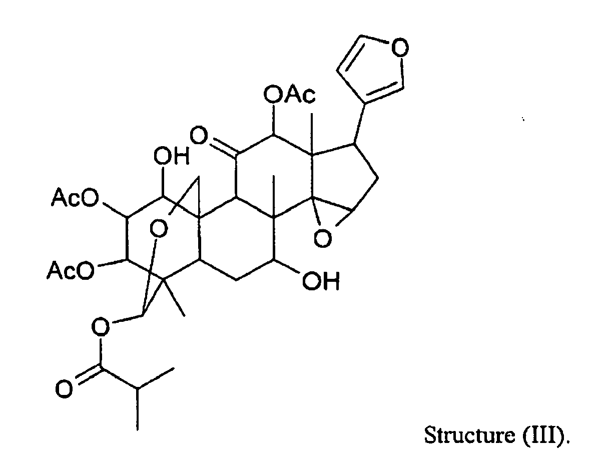

Furthermore, the pharmaceutically acceptable carrier and pharmaceutically acceptable salt are described as above.

EXAMPLE

Example 1

Extracting an effective ingredient for inhibiting hepatitis C viruses from a plant material

5.96 g of chuanlianzi (the fruit of Melia toosendan Sieb. Et Zucc.) ethanol extract was dissolved in 20 ml of a mixture of methanol and water and extracted with 15 ml of n-hexane, diethyl ether, dichloromethane and ethyl acetate for 2-3 times, respectively to obtain an n-hexane layer extraction solution, diethyl ether layer extraction solution, dichloromethane layer extraction solution and ethyl acetate layer extraction solution, respectively. Each layer extraction solution was concentrated and dried to obtain an n-hexane layer extract, diethyl ether layer extract, dichloromethane layer extract and ethyl acetate layer extract, respectively. The hepatitis C inhibition activity of each layer extract was determined by using a Huh-luc/neo-ET cell (HCV replication system) for testing thereof.

The results showed that the diethyl ether layer extract (1.113 g) had HCV replication inhibition activity. The HCV replication inhibition concentration (IC₅₀) thereof was 0.68+0.11 µg/ml.

Open column chromatography was used to isolate 1.1 g of the diethyl ether layer extract with mixtures of n-hexane and acetone with different ratios as a mobile phase and then the diethyl ether layer extract was separated to many fractions. After activity test, the selected fractions containing active ingredients were isolated by a reverse phase semi-preparative HPLC column with water and acetonitrile as a mobile phase and then two compounds were isolated. After that, the NMR spectroscopy and mass spectrometry were used to analyze the two compounds.

The NMR spectral data of the first compound showed that:

¹HNMR(500MHz, CD3OD) : 7.39 (s, 1H) ; 7.19 (s, 1H) ; 6.16 (s, 1H) ; 5.34 (s, 1H) ; 5.20 (s, 1H) ; 4.71 - 4.24(m, 5H) ; 3.80 (s, 1H) ; 3.58-3.31(m, 1H) ; 2.90 -2.54 (m, 4H) ; 2.19-1.89 (m, 8H) ; 1.37-1.33 (3H) ; 1.14-1.11 (3H) ; 0.84 (s, 3H).

The mass spectral data of the first compound showed that:

ESI⁺-MS : 621[M+2×Na]⁺ ; 598[M+Na]⁺ ; 558[M-O]⁺ , 498[M-O-OAc]⁺ ; 438[M-O-2×OAc]⁺.

Therefore, the first compound was determined as toosendanin.

Moreover, the NMR spectral data of the second compound showed that:

¹HNMR(500MHz, CD3OD) : 7.40 (s, 1H) ; 7.20 (s, 1H) ; 6.16 (s, 1H) ; 5.77 (s, 1H) ; 5.47 (s, 1H) ; 5.37 (s, 1H) ; 5.34 (s, 1H) ; 4.52 (s, 1H) ; 4.53-4.31 (m, 2H) ; 3.82 (s, 1H) ; 3.578 (m, 1H) ; 2.96-2.87 (m, 2H) ; 2.48 -2.50 (m, 1H) ; 2.20 (m, 2H) ; ; 2.08 (sm, 2H) ; 1.99 (s,3H) ; 1.93 (s,3H) ; 1.90 (s,3H) ; 1.34-1.29 (3H) ; 1.17-1.13 (m,3H) ; 0.95-0.88 (m,6H) ; 0.83 (s, 3H)

The mass spectral data of the second compound showed that:

ESI⁺-MS : 749[M+2×Na]⁺ ; 726[M+Na]⁺ ; 635[M-furan]⁺ ; 616[635-COCH(CH₃)₂]⁺ ; 558[616-OAc]⁺.

Therefore, the second compound was determined as Trichilin H.

Example 2

1. Cell cytotoxic test of toosendanin to Huh-luc/noe-ET cells

Huh-luc/neo-ET cells with concentration of 2.5×10⁴ cells/100 µl/well were seeded into a 96 well culture plate (Corning Incorporation COSTAR, 3599) and placed into a cell incubator for culturing.

The next day, a toosendanin sample was diluted with a DMEM culture medium to the concentrations of 28.73 µg/ml, 9.57 µg/ml, 3.19 µg/ml, 1.06 µg/ml, 0.35 µg/ml, 0.11 µg/ml, 0.039 µg/ml and 0.013 µg/ml, respectively, or to become the concentrations of 114.92 µg/ml, 38.33 µg/ml, 12.77 µg/ml, 4.25 µg/ml, 1.42 µg/ml, 0.46 µg/ml, 0.16 µg/ml and 0.057 µg/ml, respectively. The initial medium in the 96 well culture plate was sucked out by a vacuum pump (DOAT-704AA) without removing the cells. Then, the toosendanin culture medium with concentrations mentioned above were added into the 96 well culture plate containing cells with amount of 100 µl/well as experiment groups while untreated culture medium were added to the cells as a control group.

After culturing for two days, the medium was removed and each well containing cells of 96 well culture plate was washed twice by 100 µl of 1× PBS (1 mM KH₂PO₄, 10 mM Na₂HPO₄, 137 mM NaCl, 2.7 mM KCl) and whether there was drug precipitate on the well was observed and recorded. Then, PBS was removed and 50 µl of medium containing MTT (Sigma, M2128) with a concentration of 0.5 mg/ml was added into each well of the 96 well culture plate. After the 96 well culture plate was placed in a CO₂ culture incubator for 1 hour, DMSO (Riedel-de Haën, 60153) with amount of 150 µl/well was added into the 96 well culture plate and vortexed by a votexer (KS shaker Type 670) to thoroughly mix the resulting purple precipitate. After the purple precipitate was thoroughly mixed, the 96 well culture plate was placed in a continuous wave length microwell plate analysis system (Molecular Devices, SPECTRAMAX 190) to measure the absorbance of cells at 560 nm.

The average absorbance of the control groups was used as 100% of the cell survival rates (%) to calculate the cell survival rates (%) of the experiment groups with different concentrations of toosendanin samples. The formula of the cell survival rate was: (absorbance of experiment group/absorbance of the control group)×100%. The cell survival rates of all experiment groups were drawn to form an x-y graph and thus a trend line formula with R² value greater than 0.9 was obtained.

In the trend line formula, when y was 50, the resulting X meant 50% of the cell cytotoxic concentration (CC₅₀) or when y was 85, the resulting X meant 15% of the cell cytotoxic concentration (CC₁₅). Due to the property of the sample, CC₁₅ was calculated from the trend line formula generated by the experiment groups containing samples with concentrations of 3.19 µg/ml, 1.06 µg/ml, 0.35 µg/ml, 0.11 µg/ml and 0.039 µg/ml. The result is shown as Table 1. 50% of the cell cytotoxic concentration (CC₅₀) of the toosendanin was greater than 114.8 µg/ml, and 15% of the cell cytotoxic concentration (CC₁₅) of the toosendanin was 0.34 µg/ml.

When the cell survival rates (%) was greater than 85%, it meant that the sample at or less than the concentration (the concentration less than CC₁₅) was non-cytotoxic. The samples with non-cytotoxic concentrations were selected for a test to be performed for firefly luciferase activity ofHuh-luc/neo-ET cells (detailed in the following).

2. Estimating the HCV replication inhibition effect of toosendanin by determining the firefly luciferase activity

The Huh-luc/neo-ET cells were co-cultured with toosendanin with concentrations of 0.15 µg/ml, 0.075 µg/ml, 0.038 µg/ml, 0.019 µg/ml and 0.0085 µg/ml, respectively for 2 days and then were washed twice by 100 µl of 1× PBS (1 mM KH2PO4, 10 mM Na2HPO4, 137 mM NaCl, 2.7 mM KCl) and PBS was removed. 35 µl of 1×passive lysis buffer (Promega, E1941) was added to the cells and vortexed by a vortexer for 10 minutes to thoroughly mix the cells.

Cell suspension with an amount of 30 µl/well was added to a 96 well white plate(NUNC, 236108) for measuring luciferase activity, and 40 µl /well of luminescence analysis buffer and 20 µl /well of luminescence substrate (1 mM D-Luciferin) were sequentially added into the 96 well white plates. After the substrate was added, a microplate luminescence meter (Berthold, MPL4) was used to measure the luciferase activity (Rlu/s).

The luciferase activity of the control group was used as a standard to calculate the hepatitis C virus inhibition rates (%) of the experiment groups. The formula of inhibition rates (%) was :{[(1uciferase activity of control group)-(luciferase activity of experiment group)]/(luciferase activity of control group)}× 100%. After the sample was serial diluted and the HCV replication inhibition rates were determined at different concentrations, 50% of the HCV replication inhibition concentration (IC₅₀) was calculated by grafit5 software (Erithacus Software).

For each experiment, except for the PEG IFN alpha-2a with concentrations of 0.5 ng/ml and 0.1 ng/ml were used as positive controls for the IC₅₀ test. Additionally, cyclosporine A (CsA) with a concentration of 1 µg/ml was also used as a positive control for the IC₅₀ test.

The result is shown as Table 1, wherein 50% of the HCV replication inhibition concentration (IC₅₀) of the toosendanin was 0.045±0.004 µg/ml, and, 90% of the HCV replication inhibition concentration (IC₉₀) of the toosendanin was 0.35 µg/ml.

Example 3

1. Cell cytotoxic test of Trichilin H to Huh-luc/neo-ET cells

The experiment procedure was the same as Example 2 while the testing sample was replaced with Trichilin H. The Trichilin H sample was diluted with a DMEM culture medium to become final concentrations of 50 µg/ml, 25 µg/ml, 8.33 µg/ml, 2.78 µg/ml, 0.93 µg/ml, 0.31 µg/ml, 0.1 µg/ml and 0.03 µg/ml, respectively.

The result is shown as Table 1, wherein 50% of the cell cytotoxic concentration (CC₅₀) of the toosendanin was greater than 50 µg/ml, and 15% of the cell cytotoxic concentration (CC₁₅) of the toosendanin was 0.7 µg/ml.

2. Estimating the HCV replication inhibition effect of Trichilin H by determining the firefly luciferase activity

The experiment procedure was the same as Example 2 while the testing sample was replaced with Trichilin H. The Huh-luc/neo-ET cells were co-cultured with Trichilin H with concentrations of 0.75 µg/ml, 0.5 µg/ml, 0.25 µg/ml and 0.125 µg/ml, respectively.

The result is shown as Table 1, wherein 50% of the HCV replication inhibition concentration (IC₅₀) of the Trichilin H was 0.48±0.10 µg/ml, and 90 % of the HCV replication inhibition concentration (IC₉₀) of the Trichilin H was 0.9 µg/ml.

**Table 1: Cell cytotoxic concentration and HCV replication inhibition concentration of the test sample**

| Sample | CC₅₀ (µg/ml) | CC₁₅ (µg/ml) | IC₅₀ (µg/ml) | IC₉₀ (µg/ml) | EW (CC₅₀/IC₅₀) |
|---|---|---|---|---|---|
| Toosendanin | >114 | 0.34 | 0.045±0.004 | 0.35 | >2531.6 |
| Trichilin H | >50 | 0.7 | 0.48±0.10 | 0.9 | >104.2 |

CC₅₀: 50% of the cell cytotoxic concentration

CC₁₅: 15% of the cell cytotoxic concentration

IC₅₀: 50% of the HCV replication inhibition concentration

IC₉₀: 90% of the HCV replication inhibition concentration

EW: 50% of the cell cytotoxic concentration/50 % of the HCV replication inhibition concentration

While the invention has been described by way of example and in terms of the preferred embodiments, it is to be understood that the invention is not limited to the disclosed embodiments.

## Claims

1. Composition for use in inhibiting hepatitis C viruses or treating hepatitis C, wherein the composition comprises:
an effective amount of limonoid compound, shown as Structure (I): 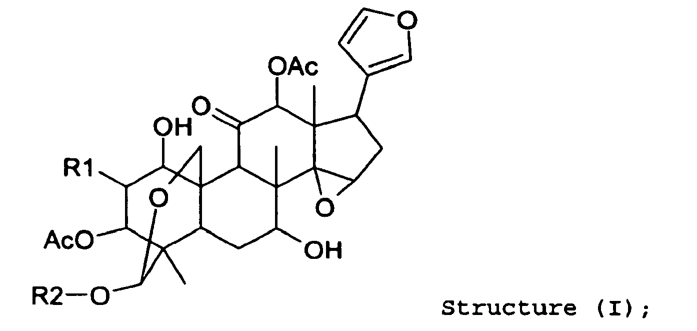 , where R1 comprises H or OAc and R2 comprises H or COCH(CH₃)₂; and
a pharmaceutically acceptable carrier or salt.

2. Composition for use as claimed in claim 1, wherein the limonoid compound is extracted from a plant material.

3. Composition for use as claimed in claim 2, wherein the plant material comprises Melia toosendan Sieb. Et Zucc. or Melia azedarach Linn.

4. Composition for use as claimed in claim 1, wherein the limonoid compound comprises toosendanin or Trichilin H, and structures of toosendanin or Trichilin H are shown as Structures (II) and (III), respectively: 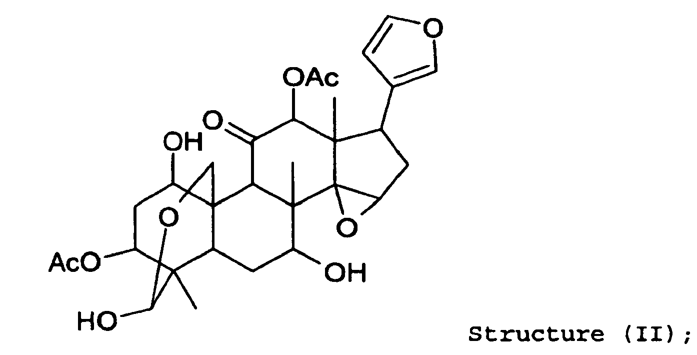 and

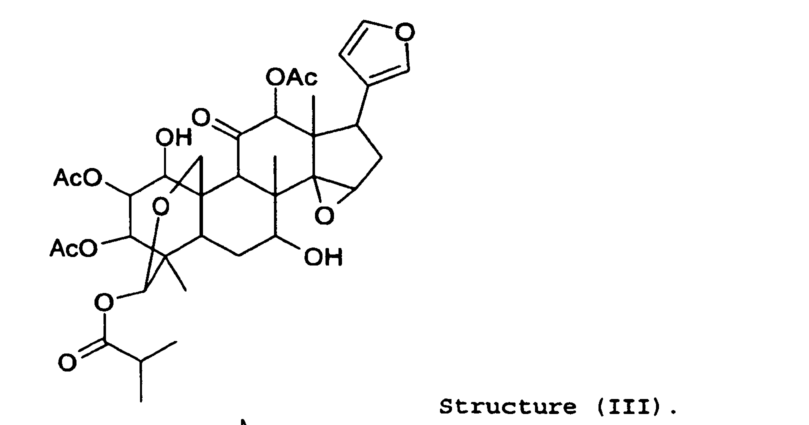

5. Composition for use as claimed in claim 1, wherein the limonoid compound comprises toosendanin and a structure of toosendanin is shown as Structure (II):

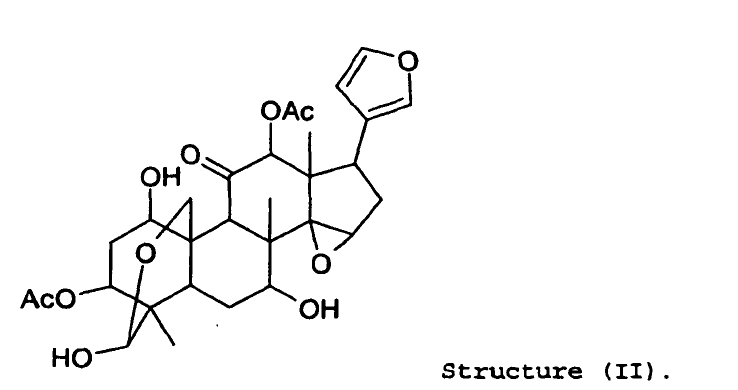

6. Composition for use as claimed in claim 5, wherein the toosendanin is extracted from a plant material.

7. Composition for use as claimed in claim 6, wherein the plant material comprises Melia toosendan Sieb. Et Zucc. or Melia azedarach Linn.

8. Composition for use as claimed in claim 1, wherein the limonoid compound comprises Trichilin H, and a structure of Trichilin H is shown as Structure (III):

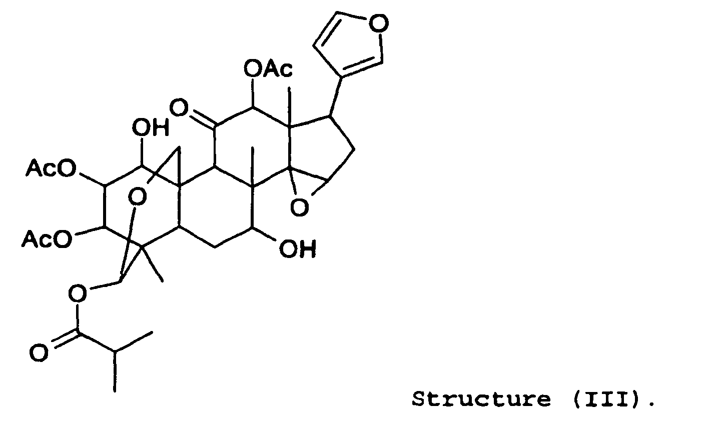

9. Composition for use as claimed in claim 8, wherein the Trichilin H is extracted from a plant material.

10. Composition for use as claimed in claim 9, wherein the plant material comprises Melia toosendan Sieb. Et Zucc. or Melia azedarach Linn.

11. Use of a limonoid compound in manufacture of a medicament for inhibiting hepatitis C viruses or treating hepatitis C, wherein the limonoid compound
is shown as Structure (I): 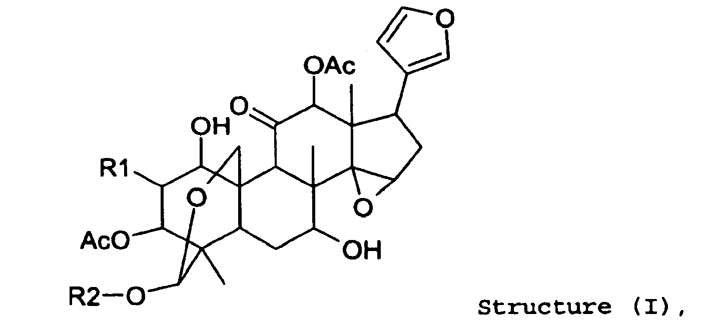 where R1 comprises H or OAc and R2 comprises H or COCH(CH₃)₂.

12. The use as claimed in claim 11, wherein the limonoid compound is extracted from a plant material.

13. The use
as claimed in claim 12, wherein the plant material comprises Melia toosendan Sieb. Et Zucc. or Melia azedarach Linn.

14. The use as claimed in claim 11, wherein the limonoid compound comprises toosendanin or Trichilin H, and structures of toosendanin or Trichilin H are shown as Structure (II) and (III), respectively: 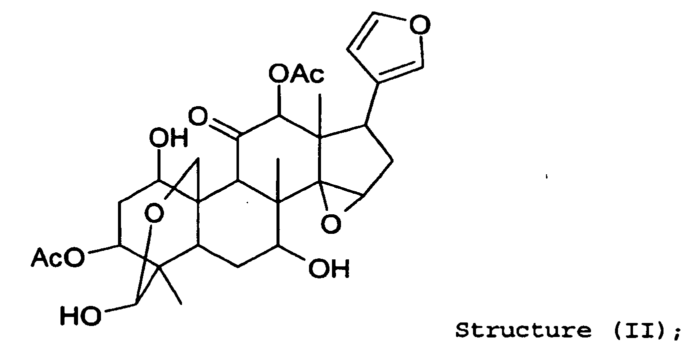 and

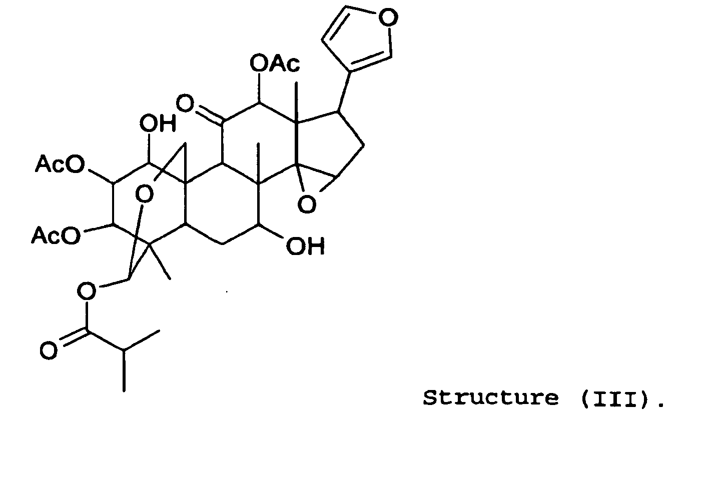

## Patentansprüche

1. Zusammensetzung zur Verwendung in der Inhibierung von Hepatitis C-Viren oder Behandlung von Hepatitis C, wobei die Zusammensetzung umfasst:
eine wirksame Menge einer Limonoidverbindung, gezeigt als Struktur (I): 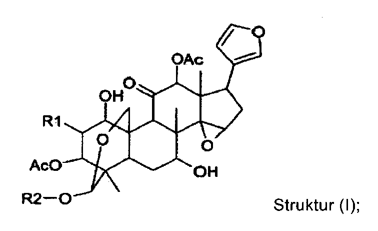
wobei R1 H oder OAc umfasst und R2 H oder COCH(CH₃)₂ umfasst; und einen pharmazeutisch verträglichen Träger oder Salz.

2. Zusammensetzung zur Verwendung wie in Anspruch 1 beansprucht, wobei die Limonoidverbindung aus einem Pflanzenmaterial extrahiert wird.

3. Zusammensetzung zur Verwendung wie in Anspruch 2 beansprucht, wobei das Pflanzenmaterial Melia toosendan Sieb. Et. Zucc. oder Melia azedarach Linn. umfasst.

4. Zusammensetzung zur Verwendung wie in Anspruch 1 beansprucht, wobei die Limonoidverbindung Toosendanin oder Trichilin H umfasst und Strukturen von Toosendanin oder Trichilin H als Strukturen (II) beziehungsweise (III) gezeigt sind: 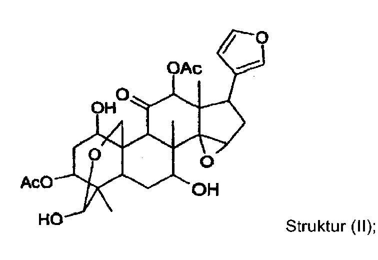 und

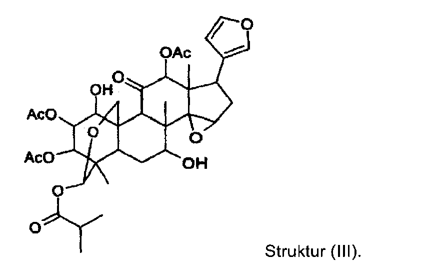

5. Zusammensetzung zur Verwendung wie in Anspruch 1 beansprucht, wobei die Limonoidverbindung Toosendanin umfasst und eine Struktur von Toosendanin als Struktur (II) gezeigt ist:

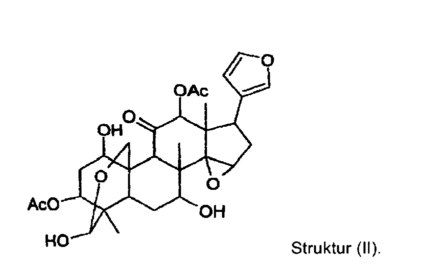

6. Zusammensetzung zur Verwendung wie in Anspruch 5 beansprucht, wobei das Toosendanin aus einem Pflanzenmaterial extrahiert wird.

7. Zusammensetzung zur Verwendung wie in Anspruch 6 beansprucht, wobei das Pflanzenmaterial Melia toosendan Sieb. Et. Zucc. oder Melia azedarach Linn. umfasst.

8. Zusammensetzung zur Verwendung wie in Anspruch 1 beansprucht, wobei die Limonoidverbindung Trichilin H umfasst und eine Struktur von Trichilin H als Struktur (III) gezeigt ist:

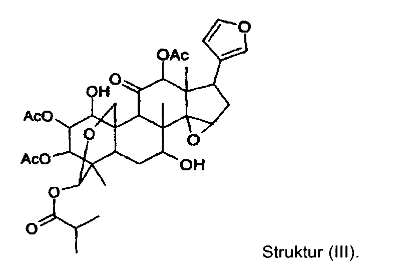

9. Zusammensetzung zur Verwendung wie in Anspruch 8 beansprucht, wobei das Trichilin H aus einem Pflanzenmaterial extrahiert wird.

10. Zusammensetzung zur Verwendung wie in Anspruch 9 beansprucht, wobei das Pflanzenmaterial Melia toosendan Sieb. Et. Zucc. oder Melia azedarach Linn. umfasst.

11. Verwendung einer Limonoidverbindung in der Herstellung eines Medikaments zur Inhibierung von Hepatitis C-Viren oder Behandlung von Hepatitis C, wobei die Limonoidverbindung als Struktur (I) gezeigt ist: 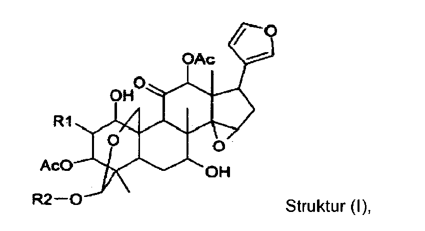 wobei R1 H oder OAc umfasst und R2 H oder COCH(CH₃)₂ umfasst.

12. Verwendung wie in Anspruch 11 beansprucht, wobei die Limonoidverbindung aus einem Pflanzenmaterial extrahiert wird.

13. Verwendung wie in Anspruch 12 beansprucht, wobei das Pflanzenmaterial Melia toosendan Sieb. Et. Zucc. oder Melia azedarach Linn. umfasst.

14. Verwendung wie in Anspruch 11 beansprucht, wobei die Limonoidverbindung Toosendanin oder Trichilin H umfasst und Strukturen von Toosendanin oder Trichilin H als Struktur (II) beziehungsweise (III) gezeigt sind: 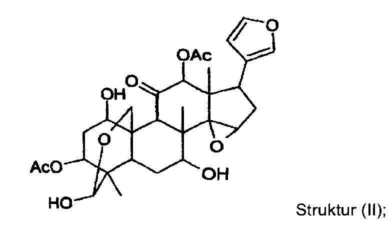 und

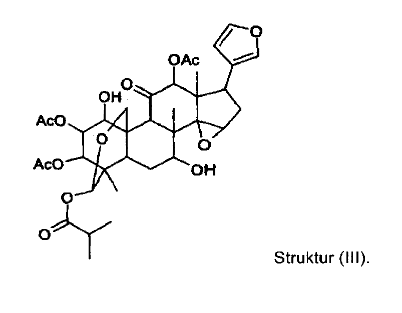

## Revendications

1. Composition pour son utilisation pour l'inhibition du virus de l'hépatite C ou le traitement de l'hépatite C, dans laquelle la composition comprend une quantité efficace de composé de type limonoïde présenté sous forme de la structure (I) : 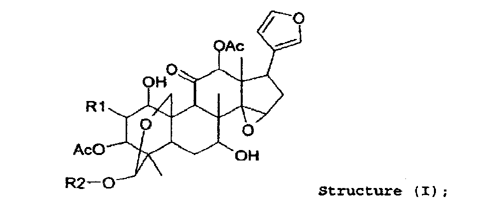 dans laquelle R1 comprend H ou OAc et R2 comprend H ou COCH(CH₃)₂ ; et
un véhicule ou un sel pharmaceutiquement acceptable.

2. Composition pour son utilisation selon la revendication 1, dans laquelle le composé de type limonoïde est extrait à partir d'une matière végétale.

3. Composition pour son utilisation selon la revendication 2, dans laquelle la matière végétale comprend Melia toosendan Sieb. Et Zucc. ou Melia azedarach Linn.

4. Composition pour son utilisation selon la revendication 1, dans laquelle le composé de type limonoïde comprend la toosendanine ou la trichiline H et les structures de la toosendanine ou de la trichiline H sont respectivement présentées sous forme de structures (II) et (III) : 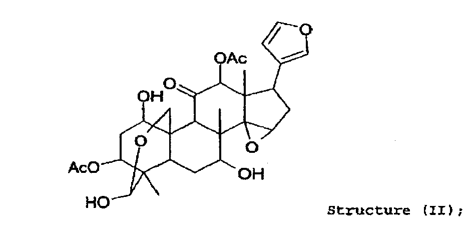 et

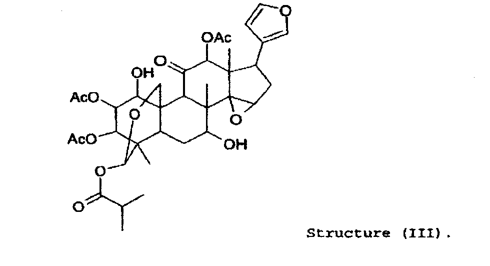

5. Composition pour son utilisation selon la revendication 1, dans laquelle le composé de type limonoïde comprend la toosendanine et une structure de la toosendanine est indiquée sous forme de la structure (II) :

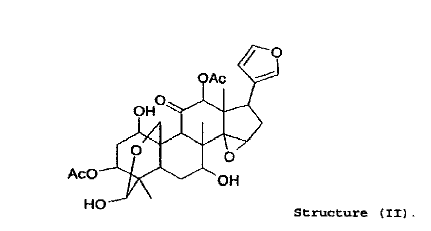

6. Composition pour son utilisation selon la revendication 5, dans laquelle la toosendanine est extraite à partir d'une matière végétale.

7. Composition pour son utilisation selon la revendication 6, dans laquelle la matière végétale comprend Melia toosendan Sieb. Et Zucc. ou Melia azedarach Linn.

8. Composition pour son utilisation selon la revendication 1, dans laquelle le composé de type limonoïde comprend la trichiline H et la structure de la trichiline H est indiquée sous forme de la structure (III) :

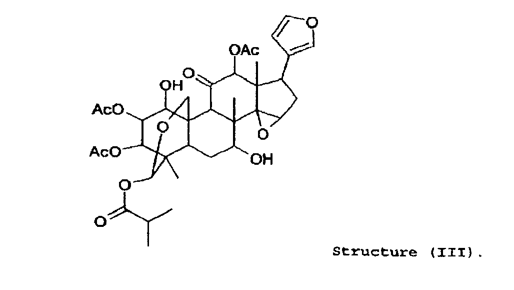

9. Composition pour son utilisation selon la revendication 8, dans laquelle la trichiline H est extraite à partir d'une matière végétale.

10. Composition pour son utilisation selon la revendication 9, dans laquelle la matière végétale comprend Melia toosendan Sieb. Et Zucc. ou Melia azedarach Linn.

11. Utilisation d'un composé de type limonoïde pour fabriquer un médicament pour l'inhibition du virus de l'hépatite C ou le traitement de l'hépatite C, dans laquelle le composé de type limonoïde est présenté sous forme de structure (I) : 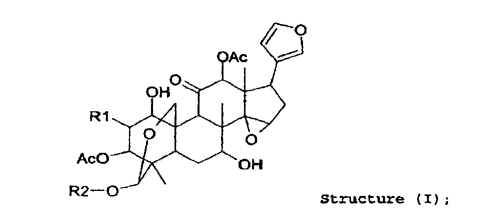 dans laquelle R1 comprend H ou OAc et R2 comprend H ou COCH(CH₃)₂.

12. Utilisation selon la revendication 11, dans laquelle le composé du type limonoïde est extrait à partir d'une matière végétale.

13. Utilisation selon la revendication 12, dans laquelle la matière végétale comprend Melia toosendan Sieb. Et Zucc. ou Melia azedarach Linn.

14. Utilisation selon la revendication 11, dans laquelle le composé de type limonoïde comprend la toosendanine ou la trichiline H et les structures de la toosendanine ou de la trichiline H sont respectivement présentées sous forme de structures (II) et (III) : 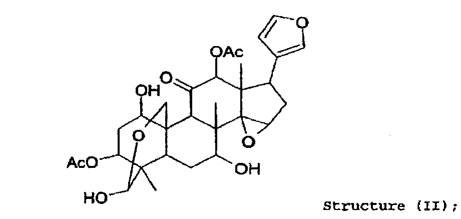 et
